Europäisches Patentamt

European Patent Office

Office européen des brevets

(19)

(11) Publication number: **0 010 438**
**B1**

(12) **EUROPEAN PATENT SPECIFICATION**

(45) Date of publication of patent specification: **09.02.83**

(21) Application number: **79302276.5**

(22) Date of filing: **19.10.79**

(51) Int. Cl.³: **B 01 D 47/12,**
**B 01 D 47/02,**
**C 07 C 47/22, C 07 C 45/78**

(54) **Apparatus for continuously removing fine dust particles from gases.**

(30) Priority: **24.10.78 JP 146166/78 U**

(43) Date of publication of application:
**30.04.80 Bulletin 80/9**

(45) Publication of the grant of the patent:
**09.02.83 Bulletin 83/6**

(84) Designated Contracting States:
**BE DE FR GB NL**

(56) References cited:
**BE - A - 545 127**
**FR - A - 2 309 277**
**NL - A - 7 611 316**
**US - A - 3 075 752**
**US - A - 4 118 313**

(73) Proprietor: **MITSUBISHI RAYON CO. LTD.**
**3-19, Kyobashi 2-chome Chuo-Ku**
**Tokyo 104 (JP)**

(72) Inventor: **Murakami, Fumiki**
**10-13, Nishisakae 2-chome**
**Otake-shi, Hiroshima-ken (JP)**
Inventor: **Ikeda, Minoru**
**908-224, Ono-cho**
**Saeki-gun, Hiroshima-ken (JP)**
Inventor: **Nakano, Toshiharu**
**9-32, Kurokawa 2-chome**
**Otake-shi, Hiroshima-ken (JP)**
Inventor: **Tokutomi, Takashi**
**2-2, Kurokawa 2-chome**
**Otake-shi, Hiroshima-ken (JP)**

(74) Representative: **Warden, John Christopher et al,**
**R.G.C. JENKINS & CO. 53/64 Chancery Lane**
**London WC2A 1QU (GB)**

Courier Press, Leamington Spa, England

Apparatus for continuously removing fine dust particles from gases

This invention relates to an apparatus for continuously removing fine dust particles from gases.

Dust collectors have heretofore been employed equipped with a venturi scrubber, a jet scrubber or glass wool to remove fine dust particles having sizes smaller than several tens of microns.

Gases obtained by catalytic oxidation reaction in the vapor phase, such as isobutylene, methacrolein, and the like contain fine dust particles of polymers e.g. of methacrylic acid.

Such gases usually have a dust concentration of about $100 \text{ mg/m}^3$. A test for removing fine dust particles using a venturi scrubber and a jet scrubber revealed a maximum removal rate of 65%. When the test was continued for several days, the throat portions and conduits for exhausting the gases were adhered and clogged with solid matter, making it impossible to continue the operation.

A dust collector employing glass wool was also tested using the same gas as used in the above test. The rate of removal of the dust particles was greater than 98%. However, the glass wool became loaded with solid matter within a short period of time, making it difficult to continue the operation.

The invention is aimed at providing a gas cleaning apparatus free of the above-mentioned defects.

According to the invention there is provided apparatus for continuously removing fine dust particles from gases with washing water, comprising at least one tray having one or more slit(s) or hole(s) with the one or at least the last tray having a baffle plate installed beneath and adjacent the slit(s) or hole(s), a gas inlet port and a water inlet port for introducing washing water onto the one or the top tray wherein the water inlet port is spaced from the slit(s) or hole(s) of the one or the top tray and wherein in the case of a plurality of trays being present, the slit(s) or hole(s) of adjacent trays are staggered on or towards alternate sides of the apparatus so that the gas and washing water travel together along the one or the top tray and are then injected together from said slit(s) or hole(s) so as to impinge upon a following tray or the baffle plate.

Embodiments of the invention are hereafter described with reference to the accompanying drawings, in which:—

Figure 1 is a vertical sectioned view of an apparatus according to this invention;

Figures 2, 3, 4 and 5 are plan views illustrating holes or slits formed in a tray:

Figure 6 is a side sectional view showing the apparatus of Figure 1 but using trays as baffle plates; and

Figure 7 is a side sectional view showing a single-stage apparatus.

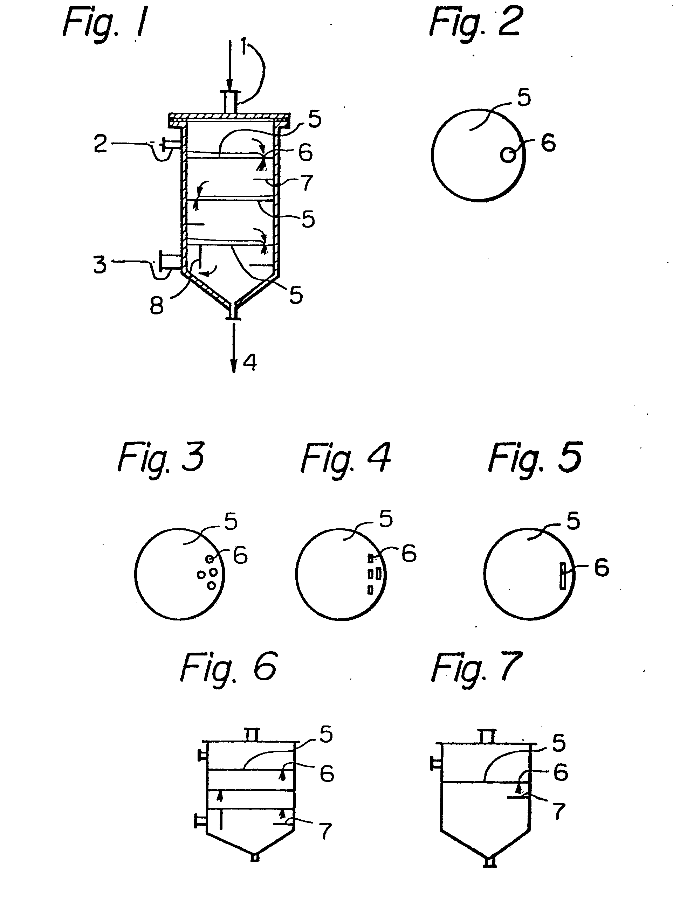

In Figure 1, a gas or gases containing fine dust particles formed e.g. by reaction in the gaseous phase, and washing water are supplied onto the tray (5) of a first stage through respective inlet ports 1 and 2. The gases and liquid travel along the tray (5) and pass through a slit or a hole (6) in the tray (5), at a distance from the water inlet port (2) at high speed and hit a baffle plate (7) where the fine dust particles are coagulated and are carried away by the water. The gas and water then pass through similar second and third stages, the slits and holes 6 being staggered on or towards alternate sides of the apparatus. The gas inlet port 1 is generally centrally over the first tray (5).

The baffle plates (7) may be substituted by the lower trays as shown in Figure 6. In putting the invention into practice, the tray or trays preferably each have a slit or a hole of such a size that the liquid-to-gas ratio L/G ranges from 1 to $50 \text{ l/m}^3$, and the gas linear velocity lies within a range of 10 to 80 m/sec.

The particles which are not removed by the tray of the first stage are removed in the second and third stages. The gases and liquid are exhausted through respective exhaust ports (3) and (4). The gas outlet port (3) is in a side of the apparatus at a distance from the hole(s) or slit(s) of the last stage tray (5) and is protected by an outlet baffle (8). The trays may be installed as a single stage or in a plurality of stages depending upon the required rate of removal of the fine dust particles.

Using the apparatus of Figure 1 equipped with three stages of trays, dust was removed from gas containing polymers of methacrylic acid. The operation was allowed to continue for six months without causing clogging to the apparatus or the conduit.

Figures 2 to 5 show examples of different possible arrangements of holes or slits formed in a tray. The number of holes or slits will be suitably determined in accordance with e.g. the concentration of dust and can be varied from stage to stage. Figure 7 illustrates another embodiment of the apparatus of the invention employing a single stage.

The apparatus of this invention is simple in construction as compared with the conventional apparatus, yet a very high rate of removal of fine dust particles is possible and the treatment can be continuously performed.

Example

A gas having a dust concentration of 100 $\text{mg/m}^3$, a temperature of 40°C and a pressure of 0.8 $\text{kg/cm}^2\text{G}$ was supplied at a rate of 5.5 $\text{m}^3/\text{hr}$, and the washing water was supplied at a rate of 38 l/hr into an apparatus having three stages of trays of an inside diameter of 54.9 mm each having a hole of a diameter of

## 

8.3 mm, with the distance between the stages being 50 mm, and with the distance between baffle plate and the tray being 25 mm. The pressure loss at the inlet port and outlet port of gas was 1200 mm $H_2O$, the particle concentration in the outlet gas was smaller than 5 mg/m$^3$, and the rate of removal of fine dust particles from the inlet gas was greater than 95%. The operation was continued for six months. There was no clogging in the apparatus or in the conduits.

Many variations are possible within the scope of the claims. For example the gas may enter through port 2 and the water through port 1, which need not be centrally situated. The gas outlet port 3 need not be in a side of the apparatus.

## Claims

1. Apparatus for continuously removing fine dust particles from gases with washing water, comprising at least one tray (5) having one or more slit(s) or hole(s) (6) with the one or at least the last tray having a baffle plate (7) installed beneath and adjacent the slit(s) or hole(s) (6), a gas inlet port (1) and a water inlet port (2) for introducing washing water onto the one or the top tray (5) wherein the water inlet port (2) is spaced from the slit(s) or hole(s) (6) of the one or the top tray and wherein in the case of a plurality of trays (5) being present, the slit(s) or hole(s) (6) of adjacent trays (5) are staggered on or towards alternate sides of the apparatus so that the gas and washing water travel together along the one or the top tray (5) and are then injected together from said slit(s) or hole(s) (6) so as to impinge upon a following tray or the baffle plate (7).

2. Apparatus according to claim 1 wherein each tray (5) comprises a baffle plate (7) installed beneath and adjacent the slit(s) or hole(s) (6).

3. Apparatus according to claim 1 or 2, wherein said trays (5) have slit(s) or hole(s) (6) of such a size that the washing water to gas ratio L/G ranges from 1 to 50 l/m$^3$ the gas has a linear velocity ranging from 10 to 80 m/sec.

4. Apparatus according to any preceding claim wherein the tray(s) (5) are generally horizontal.

5. Apparatus according to claim 4 wherein the water inlet port (2) is in a side of the apparatus over the one or the top tray (5).

6. Apparatus according to claim 5 wherein the gas inlet port (1) is generally centrally over the one or the top tray (5).

7. Apparatus according to any of claims 4 to 6 wherein the gas outlet port (3) is in a side of the apparatus at a distance from the hole(s) or slit(s) (6) of the one or the last tray (5) and is protected by an outlet baffle (8).

## Revendications

1. Appareil pour l'élimination de façon continue de particules de poussière fines dans les gaz avec une eau de lavage, comprenant au moins un plateau (5) pourvu d'une ou plusieurs fentes ou trous (6), le plateau unique ou au moins le dernier plateau comportant une tôle de chicane (7) installée au dessous de la fente ou trou ou des fentes ou trous (6) de façon adjacente à ceux-ci, un orifice (1) d'admission de gaz et un orifice (2) d'admission d'eau pour l'introduction d'eau de lavage sur le plateau unique ou le plateau de dessus (5), l'orifice (2) d'admission d'eau étant espacé de la ou des fentes ou du ou des trous (6) du plateau unique ou du plateau de dessus, et dans lequel, dans le cas de la présence d'une pluralité de plateau (5), la ou les fentes ou le ou les trous (6) des plateaux (5) adjacents sont décalés sur les côtés alternés de l'appareil ou en direction des côtés de sorte que le gaz et l'eau de lavage cheminent conjointement le long du plateau unique ou du plateau de dessous (5), et sont ensuite conjointement injectés à partir de ladite ou desdites fentes ou dudit ou desdits trous (6) de façon à être projetés sur un plateau suivant ou la tôle de chicane (7) suivante.

2. Appareil selon la revendication 1, dans lequel chaque plateau (5) comprend une tôle de chicane (7) installée au dessous de la ou des fentes ou du ou des trous (6) et de façon adjacente à ces derniers.

3. Appareil selon la revendication 1 ou 2, dans lequel lesdits plateaux (5) sont munis d'une ou plusiers fentes ou d'un ou plusieurs trous (6) ayant une dimension telle que le rapport L/G entre l'eaux de lavage et le gaz se situe entre 1 et 50 l/m$^3$, le gaz ayant une vitesse de linéaire comprise entre 10 et 80 m/sec.

4. Appareil selon l'une quelconque des revendications précédentes, dans lequel ou les plateaux (5) sont sensiblement horizontaux.

5. Appareil selon la revendication 4, dans lequel que l'orifice (2) d'admission d'eau est ménagé dans un côté de l'appareil au dessus du plateau unique ou du plateau de dessus (5).

6. Appareil selon la revendication (5) dans lequel l'orifice (1) d'admission de gaz est situé de façon générale centralement au dessus du plateau unique ou du plateau de dessus (5).

7. Appareil selon l'une quelconque des revendications 4 à 6, dans lequel l'orifice (3) de sortie des gaz est ménagé dans un côté de l'appareil à une distance du ou des trous ou de la ou des fentes (6) du plateau unique ou du dernier plateau (5) et est protégé par une chicane de sortie (8).

## Patentansprüche

1. Einrichtung zur kontinuierlichen Entfernung von feinen Staubpartikeln aus Gasen unter Zuhilfenahme von Waschwasser, mit zumindest einem Tisch (5) mit zumindest einem Schlitz oder Loch (6), wobei der eine Tisch oder zumindest der letzte Tisch eine Leit- bzw. Prallplatte (7) besitzt, die unterhalb und benachbart zu dem zumindest einen Schlitz oder Loch (6) angeordnet ist, einer Gas-Einlaßöffnung bzw. einem Gas-Einlaßstutzen (1) und einem Wasser-Einlaßstutzen (2) zur Einführung von Waschwasser auf den einen oder auf den obersten Tisch (5), wobei der Wasser-Einlaßstutzen (2) im Abstand von dem zumindest einen Schlitz oder Loch (6) des einen bzw. des obersten Tischs steht und wobei für den Fall, daß eine Vielzahl von Tischen (5) vorgesehen ist, der Schlitz oder das Loch bzw. die Schlitze oder die Löcher (6) benachbarter Tische (5) zueinander versetzt in Richtung auf die jeweils andere Seite der Einrichtung angeordnet sind, so daß das Gas und das Waschwasser zusammen entlang des einen oder des obersten Tischs (5) strömt und dann zusammen von dem Schlitz oder Loch (6) bzw. von den Schlitzen oder Löchern (6) derart eingespritzt bzw. eingeblasen wird, daß es auf einen folgenden Tisch oder auf die Prallplatte (7) prallt.

2. Einrichtung nach Anspruch 1, wobei jeder Tisch (5) eine Prallplatte (7) aufweist, die unterhalb des zumindest einen Schlitzes oder Lochs (6) und diesem benachbart installiert ist.

3. Einrichtung nach Anspruch 1 oder 2, wobei die Tische (5) zumindest einen Schlitz oder zumindest ein Loch (6) mit einer derartigen Größe besitzen, daß das Verhältnis L/G von Waschwasser zu Gas im Bereich zwischen 1 und 50 l/m³ liegt, wobei das Gas eine lineare Geschwindigkeit im Bereich zwischen 10 und 80 m/sec. hat.

4. Einrichtung nach einem der vorhergehenden Ansprüche, wobei der Tisch bzw. die Tische (5) im wesentlichen horizontal ausgerichtet sind.

5. Einrichtung nach Anspruch 4, dadurch gekennzeichnet, daß der Wasser-Einlaßstutzen (2) in einer Seite der Einrichtung über dem einen oder über dem obersten Tisch (5) vorgesehen ist.

6. Einrichtung nach Anspruch 5, dadurch gekennzeichnet, daß der Gas-Einlaßstutzen (1) im wesentlichen zentral über dem einen oder über dem obersten Tisch (5) vorgesehen ist.

7. Einrichtung nach einem der Ansprüche 4 bis 6, dadurch gekennzeichnet, daß der Gas-Auslaßstutzen (3) in einer Seite der Einrichtung und im Abstand von dem Loch oder Schlitz bzw. von den Löchern oder Schlitzen (6) des einen oder des letzten Tischs (5) angeordnet und durch eine Auslaß-Ablenkblech (8) abgeschirmt ist.

0 010 438

Fig. 1

Fig. 2

Fig. 3

Fig. 4

Fig. 5

Fig. 6

Fig. 7